Europäisches Patentamt

(19) European Patent Office

Office européen des brevets

(11) **EP 0 643 569 B1**

(12) **FASCICULE DE BREVET EUROPEEN**

(45) Date de publication et mention
de la délivrance du brevet:
**16.09.1998 Bulletin 1998/38**

(21) Numéro de dépôt: **93913074.6**

(22) Date de dépôt: **26.05.1993**

(51) Int. Cl.$^6$: **A61C 5/02**, A61B 17/32

(86) Numéro de dépôt international:
**PCT/FR93/00514**

(87) Numéro de publication internationale:
**WO 93/24071 (09.12.1993 Gazette 1993/29)**

(54) **DISPOSITIF DESTINES A ASSURER UN CONTROLE AUTOMATIQUE DU POSITIONNEMENT D'UN INSTRUMENT CHIRURGICAL**

AUTOMATISCHE KONTROLLVORRICHTUNG ZUM POSITIONIEREN EINES CHIRURGISCHEN INSTRUMENTS

DEVICE FOR AUTOMATIC VERIFICATION OF THE POSITIONING OF A SURGICAL INSTRUMENT

(84) Etats contractants désignés:
**CH DE DK ES FR GB IT LI SE**

(30) Priorité: **26.05.1992 FR 9206441**

(43) Date de publication de la demande:
**22.03.1995 Bulletin 1995/12**

(73) Titulaire: **SATELEC S.A.**
**F-33700 Merignac (FR)**

(72) Inventeur: **VALADE, Jean-François**
**F-33000 Bordeaux (FR)**

(74) Mandataire: **Bruder, Michel et al**
**Cabinet Bruder**
**46, rue Decamps**
**75116 Paris (FR)**

(56) Documents cités:
**US-A- 4 243 388**      **US-A- 4 595 019**
**US-A- 5 026 387**

EP 0 643 569 B1

**Description**

La présente invention concerne un dispositif destiné à assurer un contrôle automatique du positionnement d'un outil odontologique dans un canal radiculaire par rapport à l'apex d'une dent.

On sait qu'en odontologie le positionnement d'un instrument doit parfois être réalisé en un endroit déterminé d'un milieu donné, voisin d'un second milieu où le praticien ne peut exercer un contrôle visuel direct.

Ainsi la préparation canalaire doit répondre à deux objectifs essentiels, à savoir, d'une part, extirper le tissu pulpaire et l'évacuer du canal radiculaire et, d'autre part, usiner ce dernier pour lui donner une forme appropriée, apte à recevoir une obturation hermétique obturant parfaitement le canal jusque dans sa partie terminale.

Pour des raisons de rapidité et d'efficacité, on réalise habituellement cet usinage à l'aide d'appareils comportant un outil, tel que par exemple une lime, qui est soumis à des vibrations dont la fréquence se situe dans le domaine ultrasonore.

Un problème posé au praticien mettant en oeuvre une telle technique est d'assurer un nettoyage et un "usinage" quasi parfait du canal radiculaire de la dent du patient; tout en évitant de dépasser l'apex de celle-ci, c'est-à-dire la constriction qui se situe au fond dudit canal et qui sépare ce dernier de la muqueuse du patient, sous peine de causer à ce dernier de graves dommages.

Les moyens dont dispose le praticien pour assurer cette détection sont, d'une part, les méthodes conventionnelles de localisation, telles que la sensibilité tactile ou le contrôle radiographique et, d'autre part, les moyens de contrôle électroniques.

Ces derniers mesurent habituellement la résistance électrique existant entre une première électrode, ou électrode gingivale, mise en contact avec la gencive du patient, et une seconde électrode, parfois constituée par la lime elle-même, qui est introduite dans le canal radiculaire, une diminution de résistance au niveau de la constriction apicale permettant de signaler la proximité de la muqueuse et donc de l'apex de la dent.

On connaît, par le brevet français 2 590 476 des appareils qui permettent de surveiller, au cours de la progression d'une sonde, ou d'un outil au repos, à l'intérieur du canal radiculaire, la variation d'un paramètre physique, tel que la conductance, cette dernière subissant une augmentation brutale lorsque la sonde arrive à proximité immédiate de l'apex. Une fois la proximité de l'apex déterminée le praticien dispose alors, sur son outil, une butée repère constituée, la plupart du temps, d'une rondelle élastique qui est maintenue par frottement sur celui-ci, et qui, lors de la phase d'"usinage", lui permettra de savoir à quel moment son outil atteindra l'apex.

D'une part si un tel dispositif permet de définir avec une bonne précision la position relative de l'outil par rapport à l'apex, il nécessite pour cela la mise en place, sur l'outil, d'une butée repère. Or, le positionnement de celle-ci sur l'outil, en raison même de son mode de fixation, n'est ni précis ni fiable, dans la mesure où, par suite, par exemple, d'un faux mouvement, elle peut subir un décalage au-delà de sa position de réglage, ce qui peut entraîner, lors de la phase d'usinage, un dépassement de l'apex de la dent.

D'autre part, l'intervention se déroule alors en deux phases, à savoir une première phase de mesure et une seconde phase de réalisation de l'usinage proprement dit, ce qui implique une perte de temps aussi bien pour le praticien que pour son patient.

Enfin, dans ce type de dispositif, il arrive qu'il se manifeste une augmentation brutale de la conductivité mesurée en raison de causes diverses, sans pour autant que l'outil se trouve au voisinage de l'apex de la dent, si bien que l'on risque d'arrêter l'"usinage" du canal radiculaire avant que l'on soit arrivé à l'apex de celui-ci. Bien entendu, pour éviter cet inconvénient on peut augmenter la valeur du seuil de détection de l'apex, mais au risque alors, lors de la phase d'usinage du canal radiculaire, de continuer celui-ci au-delà de l'apex, dans la muqueuse du patient.

La présente invention a pour but de remédier à ces inconvénients en proposant un dispositif du type précité évitant l'utilisation d'une butée repère qui, éventuellement, peut être mis en oeuvre en une seule étape, et qui est insensible à une variation brusque et isolée de la conductivité.

La présente invention a ainsi pour objet un dispositif de contrôle automatique du positionnement d'un instrument d'odontologie dans un canal radiculaire par rapport à l'apex d'une dent comportant un élément conducteur apte à se déplacer dans le canal radiculaire, la muqueuse du patient, comportant des moyens de mesure de la conductance, susceptible de présenter un seuil de variation, au cours dudit déplacement, lorsque l'élément conducteur se trouve au contact de la muqueuse comportant des moyens de détection d'au moins une valeur critique prédéterminée de la conductance et des moyens de production d'un signal, lorsque ladite valeur déterminée atteint ladite valeur critique, caractérisé en ce qu'il comporte :

- des moyens de détermination de la dérivée, ou pente, de la conductance au long du déplacement de l'élément conducteur dans le canal radiculaire, et des moyens d'enregistrement permettant de mettre en mémoire la valeur de la dérivée, ou pente, maximale de la conductance, lors du déplacement de l'élément conducteur, et des moyens d'analyse permettant de déduire la valeur critique de la valeur de la dérivée, ou pente, maximale.

Dans un mode de mise en oeuvre de l'invention, l'élément conducteur est constitué d'un outil animé d'un mouvement de vibration sous l'action d'un générateur d'ultrasons, et le dispositif comporte des moyens d'asservissement permettant de commander les paramètres de fonctionnement de l'outil, à savoir son amplitude et/ou sa fréquence de vibration, les moyens d'asservissement étant aptes, lorsque la valeur de la dérivée, ou pente, de la conductance atteint ladite valeur critique, à modifier les paramètres de fonctionnement de l'outil suivant une loi prédéterminée, cette loi de fonctionnement conduisant éventuellement à l'arrêt complet de la vibration de l'outil.

On décrira ci-après, à titre d'exemple non limitatif, une forme d'exécution de la présente invention, en référence au dessin annexé sur lequel :

La figure 1 est un schéma synoptique du dispositif suivant l'invention.

La figure 2 est un diagramme représentant la courbe caractéristique de la variation de la conductance électrique au cours de la progression d'un élément conducteur dans le canal radiculaire d'une dent d'un patient.

La figure 3 est un diagramme montrant deux lois de variation dans le temps des paramètres de fonctionnement de l'outil.

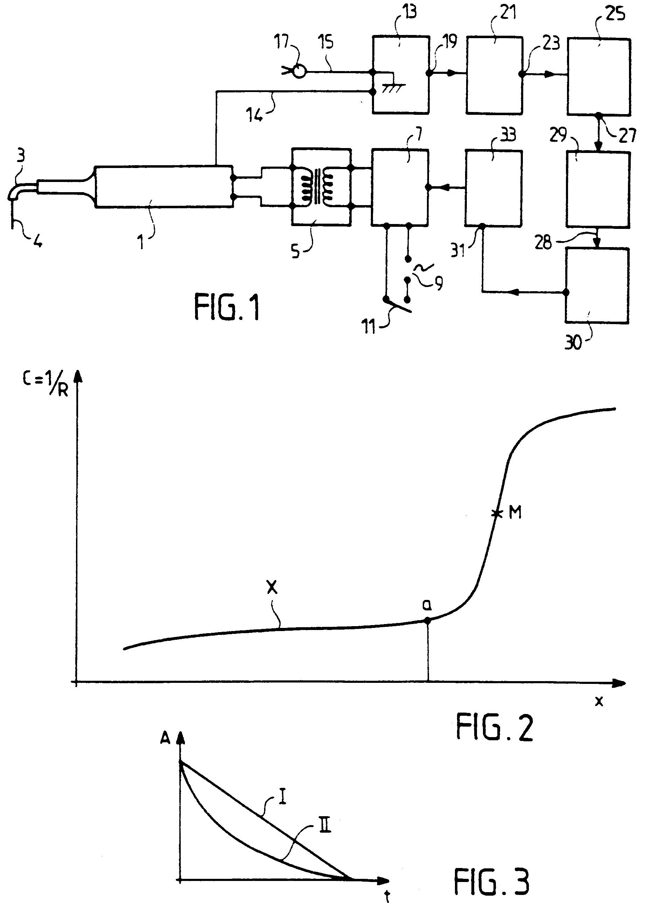

Le dispositif suivant l'invention, décrit en regard de la figure 1, est destiné à assurer la préparation d'une dent c'est-à-dire le nettoyage et l'usinage de son canal radiculaire.

Il comprend un transducteur ultrasonore 1 sur la partie avant duquel est fixée, de façon amovible et interchangeable, une sonotrode 3 pourvue d'un instrument chirurgical, à savoir dans le cas présent une lime 4. Le transducteur 1 est relié, par l'intermédiaire d'un transformateur d'isolement 5, à un générateur d'ultrasons 7 qui est lui-même relié à des moyens d'alimentation électrique 9 au travers d'un interrupteur 11 actionné par une pédale de commande.

Le dispositif comporte également des moyens permettant de réaliser la mesure de la conductance existant entre l'extrémité de la lime 4 et la muqueuse du patient. Ces moyens sont constitués d'un ohmmètre 13, dont une borne est reliée à la lime 4 par un fil conducteur 14 et l'autre borne est reliée à la muqueuse du patient par un fil conducteur 15 dont l'extrémité est pourvue d'un "clip buccal" 17. La borne de sortie 19 de l'ohmmètre 13 est reliée à un circuit d'échantillonnage 21, dont la sortie 23 est elle-même reliée à un circuit de lissage 25. La sortie 27 du circuit de lissage 25 est reliée, quant à elle, à un circuit d'analyse 29 dont la sortie 28 est reliée à un comparateur 30, assurant une comparaison du signal de sortie du circuit d'analyse 29 avec une valeur de seuil correspondant à une valeur critique prédéterminée. La sortie 31 du comparateur 30 est a son tour reliée à un circuit d'asservissement 33 assurant la commande du générateur d'ultrasons 7.

Dans ces conditions le fonctionnement du dispositif suivant l'invention s'établit comme suit :

Après avoir mis en marche le générateur d'ultrasons 7, en fermant l'interrupteur 11 au moyen de la pédale de commande, le praticien déplace la lime 4 à l'intérieur du canal radiculaire de la dent, et mesure, tout au long de ce déplacement, au moyen de l'ohmmètre 13, la résistance existant entre l'extrémité de la lime 4 et la muqueuse du patient. Le signal obtenu en sortie 19 de l'ohmmètre 13 est fourni au circuit d'échantillonnage 21 qui, dans un premier temps, inverse le signal fourni par l'ohmmètre 13 pour le transformer en un signal de conductance, puis réalise l'échantillonnage de ce signal analogique, avec une fréquence d'échantillonnage déterminée, appropriée à la précision de la mesure que l'on souhaite réaliser. Les signaux échantillonnés obtenus en sortie du circuit d'échantillonnage 21 sont ensuite lissés par le circuit de lissage 25, par exemple, par un procédé de traitement numérique, si bien, qu'en sortie 27 du circuit de lissage 25, la courbe obtenue, ou caractéristique X, (voir figure 2) représente une moyenne des mesures réalisées et donne la variation de la conductance $C=1/R$ en fonction de la profondeur de pénétration x de la lime 4 dans le canal radiculaire. Ce traitement atténue, voire même élimine totalement dans certains cas, les différents pics parasites qui, suivant la technique antérieure, étaient susceptibles de venir perturber la caractéristique X, et de signaler de façon indue la proximité de l'apex. Le signal lissé est ensuite fourni au circuit d'analyse 29 qui détermine, à chaque instant tout au long de la progression de l'outil 4 dans le canal radiculaire, la variation instantanée de la conductance C, c'est-à-dire la pente $\underline{p}$ en tous les points de la caractéristique X.

Comme représenté sur la figure 2, lorsque, au cours du déplacement de la lime 4, à l'intérieur du canal radiculaire, on se trouve en un point $\underline{a}$ de la caractéristique X, où la pente $p_a$ de la caractéristique, déterminée par le circuit d'analyse 29, est égale à la valeur critique $p_c$, à partir de laquelle on doit agir sur les paramètres de fonctionnement de l'outil, le circuit comparateur 30 émet un signal vers le circuit d'asservissement 33. Celui-ci amorce alors un processus de commande du générateur d'ultrasons 7 afin de modifier, par exemple, l'amplitude des vibrations auxquelles la lime 4 est soumise, suivant une loi de variation prédéterminée qui se termine par l'arrêt complet de la lime 4.

Cette loi de variation prédéterminée peut, par exemple, être linéaire en fonction du temps t (courbe I de la figure 3), c'est-à-dire que l'amplitude A des vibrations de la lime 4 diminue proportionnellement dans le temps pour arriver à un arrêt total des vibrations. Bien entendu une telle loi de variation dans le temps peut être autre que linéaire (courbe II de la figure 3). Elle peut également être telle qu'elle commande un arrêt immédiat des vibrations de la lime 4.

De façon intéressante, le dispositif suivant l'invention peut être pourvu de moyens de sélection permettant de choisir, parmi une série de lois préprogrammées de variations de l'amplitude des vibrations en fonction du temps, celle qui est la plus appropriée au travail à réaliser.

L'invention permet ainsi par exemple, au fur et à mesure que l'on se rapproche de l'apex, de diminuer l'amplitude des vibrations et, en conséquence, les risques liés au dépassement de celui-ci.

Bien entendu lesdites lois de variation pourront s'appliquer non seulement à la variation d'amplitude de l'outil mais également à la fréquence de ses vibrations.

On notera également que la précision du dispositif peut être améliorée en augmentant la fréquence d'échantillonnage, lorsque l'on se rapproche de l'apex, c'est-à-dire lorsque la pente de la caractéristique X atteint une certaine fraction de la pente maximale $p_M$ de celle-ci.

Dans une variante de mise en oeuvre de l'invention on peut déterminer, de façon automatique, la valeur critique prédéterminée $p_c$ de la pente. Pour cela on réalise, préalablement à l'étape d'usinage, une étape de mesure préliminaire pour laquelle on peut utiliser, en tant que sonde, soit une sonde spécifique, soit la lime 4 elle-même. Au cours de cette étape, sans alimenter le générateur d'ultrasons 7, de façon à ne pas mettre la lime en vibration, on introduit la lime 4, jouant le rôle de sonde, à l'intérieur du canal radiculaire et on la déplace à l'intérieur de celui-ci, tout en réalisant les mesures, jusqu'à ce que l'on détecte la pente maximale $p_M$ qui se situe sur la figure 2, au point <u>M</u> et, en fonction de cette pente maximale $p_M$, on détermine la pente, ou valeur critique $p_c$, soit par un décalage vers l'origine représentant la marge de sécurité que l'on entend se ménager, soit en donnant à la valeur critique $p_c$ une valeur égale à une fraction de la pente maximale $p_M$. Ainsi, une valeur critique $p_c$ égale à la moitié de la pente maximale $p_M$ de la caractéristique X permet, dans la plupart des cas, d'obtenir une détection efficace de l'apex. On pourra, de façon intéressante, lier cette valeur, au choix de la loi de variation des paramètres de fonctionnement de l'outil, par des moyens logiciels et/ou électroniques. La valeur $p_c$ est ensuite stockée dans une mémoire et l'on peut dès lors entreprendre la seconde étape du procédé, à savoir l'usinage du canal radiculaire, étant entendu que, dès que la pente de la caractéristique X atteindra la valeur critique $p_c$, le dispositif suivant l'invention mettra en oeuvre le processus de variation des paramètres de fonctionnement de l'outil c'est-à-dire de l'amplitude et/ou de la fréquence de vibration de celui-ci.

**Revendications**

1. Dispositif de contrôle automatique du positionnement d'un instrument d'odontologie dans un canal radiculaire par rapport à l'apex d'une dent comportant un élément conducteur (4) apte à se déplacer dans le canal radiculaire, vers la muqueuse d'un patient, comportant des moyens de mesure (13) de la conductance, susceptible de présenter un seuil de variation, au cours dudit déplacement, lorsque l'élément conducteur (4) se trouve au contact de la muqueuse comportant des moyens de détection (30) d'au moins une valeur critique prédéterminée de la conductance (pc) et des moyens de production d'un signal (33), lorsque ladite valeur déterminée (p) atteint ladite valeur critique (pc), caractérisé en ce qu'il comporte :

   - des moyens de détermination de la dérivée, ou pente, (p) de la conductance au long du déplacement de l'élément conducteur (4) dans le canal radiculaire, et des moyens d'enregistrement permettant de mettre en mémoire la valeur ($P_M$) de la dérivée, ou pente, maximale de la conductance, lors du déplacement de l'élément conducteur, et des moyens d'analyse permettant de déduire la valeur critique ($p_c$) de la valeur de la dérivée, ou pente, maximale ($P_M$).

2. Dispositif suivant la revendication 1 caractérisé en ce que la valeur critique ($p_c$) est sensiblement égale à la moitié de la valeur de la dérivée, ou pente, maximale ($P_M$).

3. Dispositif suivant l'une quelconque des revendications précédentes caractérisé en ce que l'élément conducteur est constitué d'un outil (4) animé d'un mouvement de vibration sous l'action d'un générateur d'ultrasons (7), et le dispositif comporte des moyens d'asservissement (33) permettant de commander les paramètres de fonctionnement de l'outil (4), à savoir son amplitude et/ou sa fréquence de vibration, les moyens d'asservissement (33) étant aptes, lorsque la valeur de la dérivée, ou pente, de la conductance atteint la valeur critique ($p_c$), à modifier les paramètres de fonctionnement de l'outil (4) suivant une loi prédéterminée, cette loi de fonctionnement conduisant éventuellement à l'arrêt complet de la vibration de l'outil (4).

**Claims**

1. Device for automatically verifying the positioning of an odontological instrument in a radicular canal with respect to the apex of a tooth, comprising a conducting element (4) adapted to move in the radicular canal towards the mucous membrane of a patient, comprising means (13) for measuring the conductance, capable of presenting a threshold of variation, during said displacement, when the conducting element (4) is in contact with the mucous membrane, comprising means (30) for detecting at least one predetermined critical value of the conductance (pc) and means for producing a signal (33) when said determined value (p) attains said critical value (pc), characterized in that it comprises:

- means for determining the drift, or slope, (p) of the conductance during the displacement of the conducting element (4) in the radicular canal, and recording means for memorizing the value $(P_M)$ of the maximum drift, or slope, of the conductance, during the displacement of the conducting element, and analysis means for deducing the critical value $(p_c)$ of the value of the maximum drift, or slope, $(P_M)$.

2. Device according to Claim 1, characterized in that the critical value $(p_c)$ is substantially equal to half the value of the maximum drift, or slope $(P_M)$.

3. Device according to either one of the preceding Claims, characterized in that the conducting element is constituted by a tool (4) animated by a movement of vibration under the action of an ultrasound generator (7), and the device comprises servo-control means (33) for controlling the operational parameters of the tool (4), namely its amplitude and/or its frequency of vibration, the servocontrol means (33) being adapted, when the value of the drift, or slope, of the conductance attains the critical value $(p_c)$, to modify the operational parameters of the tool (4) in accordance with a predetermined law, this operational law possibly leading to the complete stoppage of the vibration of the tool (4).

**Patentansprüche**

1. Vorrichtung zur automatischen Kontrolle der Positionierung eines zahnmedizinischen Instrumentes in einem Wurzelkanal bezüglich dem Scheitelpunkt eines Zahns, welche aufweist: ein leitendes Element (4), welches geeignet ist, sich im Wurzelkanal zur Schleimhaut eines Patienten hin zu verschieben, eine Einrichtung zum Messen (13) des Wirkleitwerts, welche im Verlauf des Verschiebens einen Änderungsschwellenwert liefert, wenn sich das leitende Element (4) in Kontakt mit der Schleimhaut befindet, eine Einrichtung zum Erfassen (30) von zumindest einem vorbestimmten kritischen Wirkleitwert $(p_c)$ und eine Einrichtung zum Erzeugen eines Signals (33), wenn der bestimmte Wert (p) den kritischen Wert $(p_c)$ erreicht, dadurch gekennzeichnet, daß sie aufweist:

- eine Einrichtung zum Bestimmen der Ableitung oder Steigung (p) des Wirkleitwerts entlang der Verschiebung des leitenden Elements (4) im Wurzelkanal, und eine Einrichtung zum Aufzeichnen, welche erlaubt den Wert $(P_M)$ der malen Ableitung oder Steigung der Wirkleitfähigkeit zu speichern, und eine Analyseeinrichtung, welche erlaubt, den kritischen Wert $(p_c)$ vom Wert der maximalen Ableitung oder Steigung $(P_M)$ herzuleiten.

2. Vorrichtung nach Anspruch 1, dadurch gekennzeichnet, daß der kritische Wert $(p_c)$ im wesentlichen gleich der Hälfte des Wertes der maximalen Ableitung oder Steigung $(P_M)$ ist.

3. Vorrichtung nach einem der vorstehenden Ansprüche, dadurch gekennzeichnet, daß das leitende Element aus einem Werkzeug (4) besteht, welches durch die Einwirkung eines Ultraschallgenerators (7) in eine Vibrationsbewegung versetzt wird, und die Vorrichtung eine Regeleinrichtung (33) aufweist, welche erlaubt, die Betriebsparameter des Werkzeugs (4) zu steuern, d.h. dessen Amplitude und/oder Vibrationsfrequenz, wobei die Regeleinrichtung (33) geeignet ist, wenn der Wert der Ableitung oder Steigung der Wirkleitfähigkeit den kritischen Wert $(p_c)$ erreicht, die Betriebsparameter des Werkzeugs (4) gemäß einem vorbestimmten Gesetz zu modifizieren, und dieses Betriebsgesetz eventuell zum völligen Stoppen der Vibration des Werkzeugs (4) führt.

FIG.1

FIG.2

FIG.3